Europäisches Patentamt

European Patent Office

Office européen des brevets

(11) Numéro de publication: **0 091 331**
**A1**

(12) **DEMANDE DE BREVET EUROPEEN**

(21) Numéro de dépôt: 83400473.1

(22) Date de dépôt: 08.03.83

(51) Int. Cl.³: **A 23 D 3/00**
A 23 D 5/00, A 21 D 2/16
A 61 K 7/00, B 01 F 3/08
A 23 L 1/22, A 23 C 15/16
A 23 C 11/04

(30) Priorité: 11.03.82 FR 8204082
21.02.83 FR 8302765

(43) Date de publication de la demande:
12.10.83 Bulletin 83/41

(84) Etats contractants désignés:
AT BE CH DE FR GB IT LI LU NL SE

(71) Demandeur: RHONE-POULENC SPECIALITES CHIMIQUES
"Les Miroirs" 18, Avenue d'Alsace
F-92400 Courbevoie(FR)

(72) Inventeur: Chollet, Jean
3, rue du Teil
F-79500 Melle(FR)

(72) Inventeur: Basque, Jacques
20, rue du Chemin de Fer
F-92160 Antony(FR)

(74) Mandataire: Dutruc-Rosset, Marie-Claude et al,
RHONE POULENC RECHERCHES Service Brevets Chimie et Polymères 25, Quai Paul Doumer
F-92408 Courbevoie Cedex(FR)

(54) Nouvelle préparation de sucroglycérides sous forme fluide, son procédé d'obtention et ses applications.

(57) La présente invention a trait à une nouvelle préparation de sucroglycérides sous forme fluide, son procédé d'obtention et ses différentes applications dans les domaines tels que la cosmétique, la parfumerie, la pharmacie et surtout l'industrie alimentaire.

Cette nouvelle préparation de sucroglycérides sous forme fluide comprend les sucroglycérides, une huile fluide à température ambiante et éventuellement une lécithine.

Elle peut être mise en oeuvre pour stabiliser les émulsions de composés hydrophobes dans l'eau ainsi que dans les industries de transformation des céréales et plus particulièrement les industries de cuisson des céréales.

EP 0 091 331 A1

## NOUVELLE PREPARATION DE SUCROGLYCERIDES SOUS FORME FLUIDE, SON PROCEDE D'OBTENTION ET SES APPLICATIONS

La présente invention a trait à une nouvelle préparation à base de sucroglycérides sous forme fluide, son procédé d'obtention et ses différentes applications dans les domaines tels que la cosmétique, la parfumerie, la pharmacie, et surtout l'industrie alimentaire.

Il est connu que par le terme "sucroglycérides", on désigne le mélange de produits obtenus par réaction du saccharose avec des triglycérides naturels ou de synthèse et qui contient des monoglycérides, des diglycérides, des triglycérides inaltérés (en faibles quantités), des monoesters et des diesters de saccharose en rapports variables suivant la nature du triglycéride utilisé et sa proportion mise en jeu par rapport au saccharose. Il est possible aussi que le saccharose soit combiné sous forme allant des tri- jusqu'aux octaesters. Par saccharose combiné, on entend le saccharose sous forme estérifée.

Les sucroglycérides sont doués de propriétés émulsifiantes intéressantes dues surtout aux mono- et diesters de saccharose et aux monoglycérides. Ces émulsifiants non ioniques sont totalement bio-dégradables, non toxiques, inodores, sans saveur et bien acceptés par les organismes vivants. En conséquence, ils sont utilisés dans des domaines où une certaine innocuité des produits est requise tels que la cosmétique, la parfumerie, la pharmacie et surtout dans l'industrie alimentaire animale et humaine et plus particulièrement, pour les applications suivantes : produits de la biscuiterie, biscotterie, pâtisserie, boulangerie ; produits de la confiserie et de la chocolaterie ; corps gras ; sauces et condiments, raviolis et cannellonis, gélatines, flans et entremets ; boissons sans alcool.

La mise en oeuvre des sucroglycérides pose des problèmes aux utilisateurs en raison de la forme physique sous laquelle ils se présentent. A l'état pur, ils sont sous une forme cireuse, très consistante ce qui impose de les faire fondre pour de nombreuses applications ou pour les mettre en dispersion aqueuse si c'est sous cette forme qu'ils sont ensuite employés.

On a recherché d'autres présentations de sucroglycérides de manipulation plus aisée qui consiste à les proposer sous forme pulvérulente. C'est ainsi que l'on peut citer la demande de brevet française n° 80 06463 publiée sous le n° 2 478 431 qui décrit une nouvelle préparation à base de sucroglycérides sous la forme d'une poudre qui résulte de la mise des sucroglycérides sur support constitué par un sel comestible de la caséine et une maltodextrine. Cette forme de préparation convient parfaitement bien pour certaines applications telles que l'industrie de la cuisson des céréales et tout particulièrement à la fabrication de farines composées (mixes) qui nécessite une forme pulvérulente.

Cependant, il s'est avéré au cours de la mise en oeuvre des sucroglycérides qu'il était souhaitable de disposer des sucroglycérides sous une forme fluide pour des raisons de commodités de fabrication. On trouve sur le marché, à l'heure actuelle, des sucroglycérides sous forme pâteuse assez fluide qui est le résultat d'une mise sous forme émulsionnée avec un support sucré.

Toutefois, cette présentation ne donne pas entièrement satisfaction. Outre leur mauvaise dispersabilité dans l'eau, les sucroglycérides sous cette forme sont visqueux et d'une manipulation mal aisée, en particulier ils ne peuvent pas être transvasés par gravité ou pompage à température ambiante.

Les sucroglycérides ne peuvent être sortis de leurs fûts de stockage qu'à l'aide de spatules et ne peuvent être en aucun cas déplacés par pompage. En fait, pour que les sucroglycérides puissent être versés ou pompés à une température de l'ordre de 15 à 20°C à l'aide d'une pompe de type courant telle qu'une pompe doseuse volumétrique ou une pompe à engrenages, il est nécessaire pour la bonne marche de l'appareillage que la viscosité des sucroglycérides à pomper soit généralement inférieure à 20 000 cps (20 000 mPa.s).. De plus, l'emploi de certaines pompes telles qu'une pompe centrifuge nécessite un seuil de viscosité beaucoup plus bas de l'ordre de 10 000 cps (10 000 mPa.s) (les viscosités données correspondent à des viscosités mesurées à 20°C avec un viscosimètre BROOKFIELD, modèle RVT à la vitesse de 5 tours/minute et avec une aiguille n° 6).

Le problème qui se pose est donc de trouver une nouvelle formulation des sucroglycérides qui satisfasse aux impératifs suivants :

- se présenter sous la forme d'un produit fluide susceptible d'être versé ou pompé à une température de l'ordre de 15 à 20°C sans avoir besoin d'être réchauffé au préalable ; par conséquent, avoir une viscosité inférieure à 20 000 cps (20 000 mPa.s) à une température d'environ 20°C ;

- avoir une bonne concentration en matière active de 30 à 50 % en poids mais plus généralement entre 35 et 45 % en poids ;

- être mécaniquement stable c'est-à-dire, ne pas se séparer en phases lors d'une variation de températures dans une zone se situant entre 5 et 50°C ;

- être chimiquement stable ;

- posséder une stabilité bactérienne et ne pas constituer un terrain favorable à un développement de bactéries dans les conditions de températures optimales ;

- présenter une bonne dispersabilité dans l'eau sans avoir recours à des moyens d'agitation puissants ;

- ne pas avoir d'odeur susceptible d'interférer au cours de l'application envisagée ;

- répondre aux exigences organoleptiques dans le cas d'un emploi alimentaire : présenter donc, la plus grande neutralité organoleptique possible ;

- être en conformité avec la législation du domaine d'application considéré ;

- enfin, être facile à fabriquer et d'un coût réduit.

Il a maintenant été trouvé une nouvelle préparation de sucroglycérides répondant aux exigences précitées.

La présente invention a pour objet une préparation de sucroglycérides sous forme fluide caractérisée par le fait qu'elle comprend les sucroglycérides et une huile fluide à température ambiante.

Les sucroglycérides mis en jeu selon l'invention, sont obtenus par réaction de transestérification d'un triglycéride et du saccharose.

Par triglycéride, on entend un ou plusieurs triglycérides d'acides gras aliphatiques saturés ou insaturés ayant au moins 12 atomes de préférence 14 à 20 atomes de carbone. On peut évidemment partir d'un triglycéride de synthèse obtenu par réaction du glycérol et d'acides gras mais il est plus intéressant pour des raisons économiques de faire appel aux triglycérides naturels qui sont des mélanges.

Comme triglycérides convenant à l'invention, on peut citer à titre d'exemples, le saindoux, le suif, l'huile d'arachide, l'huile de beurre, l'huile de graines de coton, l'huile de lin, l'huile de noix de coco, l'huile d'olive, l'huile de palme, l'huile de pépins de raisin, l'huile de poisson, l'huile de soja, l'huile de ricin, l'huile de coprah.

D'une manière préférentielle, on utilise des triglycérides d'acides gras ne contenant pas plus d'une double liaison et si nécessaire, on les soumet à une hydrogénation afin de réduire le nombre d'insaturations. Par conséquent, on peut employer des corps naturels hydrogénés, tels que par exemple le suif hydrogéné.

On peut donc mettre en oeuvre selon l'invention, des sucroglycérides obtenus par un procédé quelconque de transestérification.

La réaction du saccharose et de l'un des triglycérides précités peut être conduite dans un solvant réactionnel. Un des solvants le plus couramment utilisé est le diméthylformamide et l'on pourra se référer au brevet italien n° 650 389 qui propose de faire la réaction dans ce solvant, en présence de carbonate de potassium anhydre à une température d'environ 95°C. On a préconisé l'emploi d'autres solvants tels que la pyridine (brevet américain n°2831855), les dérivés de la morpholine ou de la pipéridine (brevet américain n° 2 831 856), le diméthylsulfoxyde (brevet américain n° 2 812 324) mais l'on ne perdra pas de vue que ces différents solvants organiques y compris le diméthylformamide ne peuvent être utilisés que dans la mesure où l'on obtient des sucroglycérides adaptés à l'application envisagée et que dans le cas où ils sont destinés à un usage alimentaire, ils ne doivent pas contenir d'impuretés gênantes d'un point de vue physiologique ce qui peut commander des opérations ultérieures de purification. Par exemple, dans le cas d'un procédé au diméthylformamide, la teneur résiduelle en solvant doit être inférieure à 5 p.p.m.

On peut également faire appel à des sucroglycérides obtenus selon des procédés sans solvant qui font appel soit à une fine dispersion de saccharose (certificat d'addition de brevet français n° 2 029 508 -69/15 314) soit à une haute température telle que 190°C (brevet français n° 2 047 603 - 70/17 075), soit à de longs temps réactionnels d'environ 10 à 20 heures (brevet français n° 2 221 436 - 74/08 639).

Dans tous les cas, ces procédés devront être adaptés afin d'obtenir des sucroglycérides convenant à l'invention. Plus particulièrement, on utilisera les sucroglycérides obtenus selon un procédé parfaitement bien approprié à des fins alimentaires et qui est décrit dans la demande de brevet français 79/20 758 publiée sous le n° 2 463 512. Ledit procédé de préparation de sucroglycérides par transestérification du saccharose et d'un triglycéride est caractérisé par le fait que l'on effectue une alcoolyse limitée du triglycéride avant réaction avec le saccharose. Selon un mode de réalisation préférentiel de l'invention, on fait réagir un triglycéride et l'éthanol en présence de carbonate de potassium à la température de reflux sous pression atmosphérique pendant 2 heures - 2 heures 30, puis l'on introduit le saccharose et le carbonate de potassium en 30 minutes environ dans le milieu réactionnel contenant déjà un savon de potassium, sous agitation et à une température de 120°C - 130°C, l'ensemble étant maintenu sous atmosphère de gaz inerte et l'on élève la température entre 130°C - 135°C tout en établissant une pression réduite d'environ 150 mm de mercure et l'on prolonge le chauffage jusqu'à obtention d'une durée de réaction de 4 à 5 heures. Le mélange obtenu est soit coulé et utilisé tel quel, soit neutralisé plus ou moins fortement à l'acide par exemple, acétique, phosphorique, citrique ou lactique. Il peut être purifié suivant les procédés usuels.

Quel que soit le procédé d'obtention des sucroglycérides utilisé, il est nécessaire le plus souvent de procéder à une étape de purification : on peut faire appel dans cette optique aux méthodes connues de l'art antérieur (brevet français n° 1.338.709 - PV n°7.567 et brevet français n° 1 350 654 - PV n° 7.515).

D'une manière préférentielle, on emploie aux fins de l'invention, les sucroglycérides d'huile de palme, de saindoux, d'huile de coprah, de suif. Ils se présentent sous forme de pâtes plus ou moins consistantes et se différencient commercialement par leur point de fusion :

- Sucroglycérides de saindoux ............ 47 à 50°C
- Sucroglycérides de suif ................ 50 à 55°C
- Sucroglycérides d'huile de palme ....... 55 à 58°C
- Sucroglycérides d'huile de coprah ...... 60 à 62°C

Encore plus préférentiellement, on choisit les sucroglycérides d'huile de palme ou de suif.

Conformément à la présente invention, les sucroglycérides sont combinés à une huile fluide selon un procédé décrit ultérieurement.

L'huile susceptible d'être utilisée selon l'invention doit être fluide à température ambiante c'est-à-dire, que sa température de solidification doit être inférieure à la température ambiante. On entend par température ambiante une zone de température variant de 10 à 25°C, mais le plus souvent comprise entre 16 et 22°C. La borne supérieure de l'intervalle de température précité ne présente évidemment aucun caractère critique.

Comme huiles convenant aux préparations de l'invention, on peut citer : l'huile d'arachide, l'huile de graines de coton, l'huile de noix, l'huile d'olive, l'huile de navette, l'huile de pépins de raisin, l'huile de moutarde, l'huile de soja, l'huile d'oeillette, l'huile de ricin, l'huile d'amandes douces ou amères, l'huile de poisson, l'huile de tournesol, l'huile de colza, l'huile de maïs, l'huile de vison, l'huile de sésame, l'huile de pied de boeuf ou leurs mélanges.

D'une manière préférentielle, on fait appel aux huiles suivantes : l'huile de colza, l'huile d'arachide, l'huile de maïs, l'huile de tournesol, l'huile d'oeillette, l'huile de graines de coton, l'huile de soja.

Le choix de l'huile intervenant dans les préparations de l'invention sera fait en fonction de l'application envisagée. On sélectionnera évidemment, une huile comestible dans le cas d'un emploi ultérieur alimentaire.

Les nouvelles préparations à base de sucroglycérides selon l'invention combinent les deux constituants précités en proportions qui peuvent varier dans de larges limites.

D'une manière préférentielle, les pourcentages pondéraux de chaque constituant seront choisis dans les limites suivantes :

- de 30 à 50 % de sucroglycérides
- de 50 à 70 % d'une huile fluide.

On donne ci-après, les compositions préférées des préparations de l'invention :

- de 35 à 45 % de sucroglycérides
- de 55 à 65 % d'une huile fluide.

Poursuivant ses recherches, la demanderesse a constaté que dans le cas de certains sucroglycérides, en particulier, les sucroglycérides de suif, l'addition d'huile permettait de faire diminuer la viscosité d'une manière significative mais n'était pas suffisante pour se trouver dans la zone de viscosité préférentielle pour une température d'utilisation de l'ordre de 15 à 20°C.

Il a été trouvé que les résultats obtenus étaient le plus souvent supérieurs lorsque l'on adjoignait un troisième additif tel qu'une lécithine.

La présente invention a également pour objet une préparation améliorée sous forme fluide caractérisée par le fait qu'elle comprend les sucroglycérides, une lécithine et une huile fluide à température ambiante.

La lécithine intervenant dans ces préparations améliorées est un produit connu mais complexe : c'est un phospholipide ou phospha- tide qui résulte de la combinaison d'esters des acides oléiques, stéariques, palmitiques avec l'acide glycéro-phosphorique et la choline.

Selon l'invention, on peut faire appel aux lécithines commercialisées sur le marché qui peuvent être d'origine animale ou d'origine végétale. La lécithine animale est extraite notamment, du jaune d'oeuf et la lécithine végétale provient, généralement, des graines de soja. Cette dernière est la plus couramment employée et est choisie préférentiellement selon l'invention.

Les préparations améliorées à base de sucroglycérides selon l'invention combinent les trois constituants précités en proportions qui peuvent aussi varier dans de larges limites.

La proportion de lécithine représente de 2 à 30 % du poids du mélange constitué par les sucroglycérides et la lécithine et de préférence de 20 à 30 %.

D'une manière préférentielle, les pourcentages pondéraux de chaque constituant seront choisis dans les limites suivantes :

- de 30 à 70 % du mélange constitué par les sucroglycérides et la lécithine
- de 70 à 30 % d'une huile fluide.

On donne ci-après, les compositions préférées des préparations améliorées de l'invention :

- de 40 à 60 % du mélange constitué par les sucroglycérides et la lécithine
- de 60 à 40 % d'une huile fluide.

Le mode d'obtention des préparations fluides à base de sucroglycérides selon l'invention et celui des préparations améliorées est identique à la différence près de l'introduction supplémentaire de la lécithine.

Un autre objet de la présente invention est le procédé de fabrication desdites préparations qui consiste à faire fondre les sucroglycérides, à procéder au mélange des sucroglycérides fondus, de l'huile fluide et éventuellement de la lécithine, à homogénéiser le mélange par une douce et courte agitation qui est ensuite interrompue, à refroidir le mélange obtenu à une température d'au plus 22°C, à soumettre le mélange refroidi à une douce agitation avant son conditionnement.

L'ordre d'introduction des constituants des préparations n'est pas critique et l'on peut envisager de nombreuses variantes.

Dans tous les cas, le procédé contient une étape qui consiste à faire fondre les sucroglycérides à une température comprise entre 60° et 80°C et de préférence entre 70° et 75°C. Au cours de cette opération il peut être souhaitable d'agiter doucement la masse à réchauffer afin d'assurer une bonne répartition de la température.

Dans les sucroglycérides fondus, on introduit éventuellement la lécithine et dans le mélange obtenu, on ajoute l'huile qui est à température ambiante pouvant varier entre 10° et 25°C mais, le plus généralement, entre 16° et 22°C. Cet ordre d'introduction peut être inversé et l'on peut incorporer sans inconvénient dans l'huile, le mélange de sucroglycérides fondus et de lécithine.

On peut également additionner éventuellement la lécithine simultanément à l'huile dans les sucroglycérides fondus ou inversement.

Dans l'exposé qui suit de la présente invention, on détaillera les conditions de mise en oeuvre de la première variante qui restent valables pour les autres.

Dans les sucroglycérides maintenus à une température entre 60 et 80°C, on introduit éventuellement progressivement et sous agitation la lécithine qui se trouve sous une forme sirupeuse. Ensuite, l'incorporation de l'huile dans ce mélange se fait, progressivement, sous agitation en l'additionnant par fractions ou en continu.

La durée de cette mise en oeuvre dépend des capacités de production de l'appareillage et peut varier, par exemple, entre 15 et 30 minutes.

Au cours de cette introduction, il est nécessaire d'agiter le mélange afin d'obtenir un mélange homogène. On a constaté qu'il était préférable d'agiter le mélange, au minimum, afin d'éviter d'y inclure de l'air qui risque de provoquer la formation des mousses.

Par conséquent, on additionne l'huile dans les sucroglycérides fondus éventuellement associés à de la lécithine sous agitation qui sera interrompue dès qu'il y a apparition de mousses.

Les conditions d'agitation doivent être douces. La vitesse d'agitation dépend du type d'agitateur et du rapport du diamètre de l'agitateur et de la cuve. A titre d'exemple, on fixe la vitesse pour un agitateur à cadre qui passe très près des parois de 10 à 20 tours/minute pour une cuve de 1 m de diamètre.

En fin d'addition de l'huile, on homogénéise le mélange par une agitation d'une durée de 1 à 15 minutes mais de préférence de 2 à 5 minutes.

La vitesse d'agitation doit être lente : elle est donc du même ordre de grandeur que donnée précédemment.

La température du mélange tombe aux environs de 45 à 55°C.

Une fois que le mélange obtenu présente un aspect homogène, on réalise l'étape de refroidissement à une température inférieure de 22°C mais située de préférence entre 15 et 20°C. La borne inférieure définissant la gamme de température n'est pas critique et peut atteindre des valeurs aussi faibles que 5°C.

Par contre, on a constaté de façon surprenante que la limite supérieure de la température de refroidissement était critique et devait être inférieure à 22°C. Si la température est choisie plus élevée, par exemple 25°C, on obtient une préparation qui ne possède pas les caractéristiques requises de fluidité dans le cas d'une baisse de température lors de son utilisation.

Le refroidissement du mélange ne doit pas être brutal afin de ne pas conduire à la formation d'agglomérats. Il peut être effectué en laissant la masse se refroidir à température ambiante ou accéléré par circulation d'un liquide de refroidissement dans la double enveloppe de la cuve du mélange, le plus couramment de l'eau froide à une température de 3 à 15°C.

La durée de cette étape de refroidissement dépend évidemment de la masse à refroidir ; donc, de la capacité de production de l'appareillage. Généralement, on compte entre 2 et 4 heures pour 1 000 kg de masse à refroidir.

Au cours du refroidissement, il peut y avoir lieu de remettre l'agitation par intermittence, de manière à égaliser la température dans la masse du mélange. Par exemple, une agitation de 30 secondes par demi-heure ou 1 minute par heure est souhaitable.

Quand la température souhaitée du mélange refroidi est atteinte, on procède à une nouvelle agitation qui a pour but de rendre le mélange fluide. Cette agitation finale n'a pas de durée critique mais le plus souvent de 2 à 15 minutes suffisent. Pour des raisons économiques, il n'y a pas lieu de poursuivre l'agitation pendant une durée supérieure à environ 5 minutes. Sa durée préférentielle est de 2 à 5 minutes.

On a remarqué de manière inattendue que cette agitation conférait à la préparation de l'invention une fluidité et cet état était irréversible et que si l'on supprimait cette agitation, on obtenait une masse compacte. On suppose donc et cette interprétation n'a aucun caractère limitatif de la portée de notre invention que le fait d'agiter le mélange, doit rompre son édifice cristallin et conduit à une préparation fluide, sans aucun phénomène de réversibilité.

Les préparations ainsi obtenues peuvent alors être conditionnées, habituellement dans des fûts, par exemple à ouverture totale. Dans le cas d'un stockage prolongé, il est préférable de les mettre à l'abri de l'air, sous couverture de gaz inertes tels que l'azote.

La fabrication des préparations de l'invention est conduite dans un appareillage classique qui ne présente donc aucune particularité.

L'opération de la fonte des sucroglycérides peut être réalisée au bain-marie ou dans une cuve à double enveloppe où l'on injecte de la vapeur soufflante. On peut également utiliser un fondoir dont la particularité est d'être parfaitement adapté pour réaliser la fonte des sucroglycérides du fait qu'il assure une excellente répartition de la chaleur dans la masse à réchauffer et évite les points de surchauffage et qui présente l'avantage d'être facile à nettoyer, entretien rendu le plus souvent difficile de par la nature des sucroglycérides.

Il est préférable de prévoir un moyen d'agitation mécanique tel qu'un agitateur à ancre ou à cadre.

Le mélange des sucroglycérides et de l'huile peut être réalisé dans une cuve de préparation ou dans le même appareillage que l'étape précédente dans la mesure où ils sont équipés d'un moyen d'introduction de l'huile, d'un moyen d'agitation et d'une double enveloppe permettant d'assurer le refroidissement par circulation d'un liquide de refroidissement.

Le dispositif d'addition de l'huile ou des sucroglycérides selon le cas est de nature quelconque et l'on peut utiliser par exemple, une pompe centrifuge, une pompe doseuse volumétrique.

Comme types d'agitateurs on peut faire appel aux agitateurs rotatifs lents tels que l'agitateur à cadre, l'agitateur à ancre, l'agitateur à pâle droite simple, les agitateurs à plaque verticale pleine ou perforée.

Dans le cas où l'on additionne de la lécithine, cette dernière peut être introduite dans les sucroglycérides fondus, par l'intermédiaire d'un dispositif d'addition soit·au niveau du fondoir ou soit au niveau de la cuve de préparation en même temps que l'huile.

Conformément à la présente invention, on obtient des préparations de sucroglycérides dont les caractéristiques dépendent de la nature des sucroglycérides mis en jeu et de la présence ou non de lécithine.

La demanderesse a trouvé que les préparations de sucroglycérides d'huile de palme n'avaient pas besoin de contenir de lécithine pour présenter de bonnes caractéristiques qui sont les suivantes :

- elles se présentent sous la forme d'une dispersion de sucroglycérides dans l'huile pouvant aller jusqu'à la solution ce qui dépend de la concentration en sucroglycérides, de la nature de l'huile et de la température de la préparation ;

- elles sont fluides dans une zone d'utilisation de 15 à 20°C et peuvent donc être facilement transvasées par gravité ou par pompage ;

- leur concentration en matière active est bonne de 30 à 50 % mais le plus souvent entre 35 et 45 % en poids ;

- leur stabilité mécanique est bonne : elles peuvent supporter des variations de températures entre 5 et 50°C : il y a changement d'état sans séparation de phases. Elles peuvent passer sans inconvénient de l'état pâteux à l'état liquide et inversement ;

- elles sont stables chimiquement : il n'y a pas d'interaction de leurs constituants ;

- elles ne présentent aucun risque de développement bactérien du fait qu'elles sont anhydres ;

- elles possèdent de très bonnes propriétés de dispersabilité dans l'eau ;

- elles sont d'une couleur jaune clair et sont pratiquement inodores ;

- elles peuvent être mises aisément en conformité avec la législation du domaine d'application considéré.

Par contre s'agissant des sucroglycérides de suif, la demanderesse a constaté que l'addition d'huile permettait d'abaisser, d'une manière significative, leur viscosité mais que le résultat obtenu n'était pas encore satisfaisant lorsque l'on travaillait à température ambiante.

Donc pour obtenir des caractéristiques équivalentes à celles des préparations de sucroglycérides d'huile de palme, il parait nécessaire d'adjoindre la lécithine dans la préparation.

Les préparations améliorées de sucroglycérides se distinguent des préparations sans lécithine par les points suivants :

- elles sont d'une couleur jaune-brun ;

- elles présentent une fluidité améliorée pour une même teneur en huile ;

- leur concentration en matière active constituée par les sucroglycérides et la lécithine est supérieure et peut aller jusqu'à 70 % mais le plus souvent se situe entre 40 et 60 % en poids.

Par conséquent, en présence de sucroglycérides de nature quelconque, on propose selon l'invention pour les rendre fluides, d'additionner dans un premier temps, uniquement l'huile fluide.En cas de résultats insuffisants, on préconise d'ajouter de la lécithine.

Les sucroglycérides sont utilisés en raison de leurs propriétés tensio-actives dans le domaine alimentaire, en cosmétique, en parfumerie et comme auxiliaires en pharmacie car ils facilitent la dispersion dans l'eau des composés hydrophobes et stabilisent les émulsions obtenues.

Par composés hydrophobes, on entend, par exemple, les huiles essentielles du domaine alimentaire ; les corps gras alimentaires, margarine, beurre, beurre basses calories, les compositions de laits synthétiques pour l'alimentation des jeunes animaux notamment les veaux ; les huiles essentielles utilisées en parfumerie et cosmétique et les corps gras intervenant dans les différents produits de beauté et d'hygiène.

Par ailleurs, les sucroglycérides sont employés dans l'industrie de la cuisson des céréales car ils confèrent à la pâte d'autres propriétés en plus de celles d'améliorer la stabilité des émulsions de matières grasses.

Les préparations de l'invention peuvent être mises en oeuvre dans les applications habituelles des sucroglycérides.

Dans l'industrie alimentaire, on destine généralement les sucroglycérides d'huile de palme à l'alimentation humaine et les sucroglycérides de suif à l'alimentation animale.

Dans le domaine alimentaire, on peut utiliser les sucroglycérides de l'invention pour la préparation d'émulsions ou de dispersions d'huiles essentielles en vue d'obtenir des concentrats liquides ou des préparations destinées à l'atomisation sur support qui seront ensuite introduites pour aromatiser les produits alimentaires et plus spécialement les boissons sans alcool.

On entend par "huiles essentielles", des mélanges de divers produits plus ou moins volatils issus d'espèces végétales par un procédé physique approprié tel que la distillation effectuée dans un courant de vapeur d'eau, l'enfleurage, la macération, l'extraction aux solvants et l'expression. On les appelle également "essences".

Comme exemples d'huiles essentielles, on peut citer : l'essence d'absinthe, l'essence de racines d'angélique, l'essence d'anis, l'essence de cannelle, l'essence de citronnelle, l'essence de coriandre, l'essence d'amande amère, l'essence de genièvre, l'essence de gingembre et toutes les essences d'agrumes comme les citrons, les mandarines, les oranges, les pamplemouses etc ...

Sous la dénomination d'huiles essentielles, on désigne également les "résinoïdes", qui sont les résidus huileux aromatiques résultant de la concentration de l'extrait aromatique obtenu par évaporation du solvant (notamment l'alcool) dans lequel a eu lieu la macération de plantes ou graines telles que laurier, muscade, paprika, thym, vanille, etc ....

La liste des huiles essentielles établie ci-dessus n'est pas limitative. On pourra se reporter pour la compléter à l'encyclopédie "ENCYCLOPEDIA CHEMICAL TECHNOLOGY - KIRK OTHMER - Volume 14, OILS, ESSENTIAL et Volume 9 FLAVORS AND SPICES".

La mise en oeuvre des sucroglycérides de l'invention facilitent la mise en émulsion des huiles essentielles.

La préparation des émulsions d'huiles essentielles dans l'eau stabilisées par les préparations de sucroglycérides de l'invention peut être réalisée selon deux modes d'exécution qui consistent :

- soit à faire une dispersion de sucroglycérides dans l'eau puis à y introduire l'huile essentielle,

- soit à faire une dispersion d'huile essentielle dans l'eau puis à y ajouter les sucroglycérides.

La voie à suivre dépend de l'origine de l'huile essentielle et ne peut être déterminée qu'après expérimentation. Par exemple, on peut mentionner que les émulsions stabilisées d'essences d'agrumes et d'anis sont obtenues en suivant la deuxième technique opératoire.

Les préparations de l'invention peuvent stabiliser des émulsions dans l'eau d'huiles essentielles à une concentration représentant jusqu'à 15 à 20 % du poids total de l'émulsion. La borne inférieure ne présente aucun caractère critique.

La quantité de préparations de sucroglycérides de l'invention à utiliser exprimée en poids de sucroglycérides représente de 40 à 60 % du poids de l'huile essentielle à disperser. On peut mettre des quantités plus fortes allant jusqu'à 100 % mais cela n'amène aucun avantage au niveau de la stabilité de l'émulsion.

L'eau utilisée comme milieu de dispersion peut être de l'eau de ville ou de l'eau permutée. Elle est en quantité suffisante pour compléter l'émulsion à 100 % en poids.

La confection des émulsions est réalisée, simplement par addition des sucroglycérides ou de l'huile essentielle au milieu aqueux maintenu à une température de 60 à 80°C et mis sous bonne agitation par des moyens classiques d'agitation (agitateur à turbine ...). Une fois la dispersion des sucroglycérides ou de l'huile essentielle obtenue, on ajoute progressivement et sous agitation selon le cas, l'huile essentielle ou les sucroglycérides. Il y a dispersion simultanée du produit ajouté. Sa durée d'addition dépend du volume final de l'émulsion.

Les émulsions d'huiles essentielles sont très bien stabilisées par les préparations de sucroglycérides de l'invention.

D'une manière similaire, on peut également stabiliser avec les préparations de sucroglycérides de l'invention, les émulsions grasses alimentaires : corps gras alimentaires, beurre, margarine, beurre basses calories, etc...

Dans ce cadre, une application particulièrement importante desdites préparations réside dans la confection d'émulsions stables telles que les compositions de laits synthétiques pour l'alimentation des jeunes animaux notamment les veaux.

Le lait de réengraissement pour veaux est un lait dans lequel on a enlevé la matière grasse noble pour la remplacer par du suif. Il est livré à l'utilisateur sous forme de poudre de lait et de suif représentant de 15 à 35 % du poids du lait: le suif pouvant être partiellement substitué par des huiles de poisson.

Lors de son emploi, l'utilisateur doit reconstituer le lait par addition d'eau.

Ainsi la mise en oeuvre des préparations de l'invention en particulier des sucroglycérides du suif permet à la poudre de lait contenant du suif de se mettre facilement en solution et l'émulsion de matière grasse est fine et homogène : il y a très peu de mousses et le phénomène de barattage qui correspond à la formation d'agglomérats de la matière grasse est rarement observé.

La poudre de lait de réengraissement faisant intervenir les préparations de l'invention peut être obtenue selon les procédés usuels du domaine considéré. Une technique classique consiste à sécher par atomisation simultanée d'une part, du lait maigre et d'autre part du suif amené à l'état fondu vers une température de l'ordre de 40°C à 60°C dans lequel on a introduit la préparation de l'invention.

Une préparation préférée pour ce type d'application comprend des sucroglycérides de suif, de la lécithine de soja et de l'huile de soja ou de l'huile de colza.

La quantité de préparation de l'invention mise en jeu exprimée en poids de sucroglycérides représente de 1 à 3 % du poids du suif.

La quantité de suif représente de 15 à 35 % du poids de lait maigre.

La reconstitution du lait se fait, sous agitation, par addition d'eau chaude dont la température varie de 40 à 60°C en une quantité telle que le lait obtenu contienne de 20 à 25 % de matière grasse.

Cet aliment d'allaitement possède toutes les propriétés recherchées.

Il est à noter que le développement des sucroglycérides dans l'alimentation animale s'est trouvé souvent limité du fait qu'il fallait refondre les sucroglycérides vers 75-80°C lors de leur emploi ou qu'il fallait les transporter à l'état fondu dans des camions citernes calorifugés. La présentation des sucroglycérides sous la forme de préparations fluides selon l'invention permet de les introduire aisément dans le suif fondu.

Les préparations de sucroglycérides de l'invention peuvent également être employées en parfumerie, en cosmétique en raison de leur fonction d'agent émulsifiant pour la dispersion des huiles essentielles contenant les principes odoriférants et la stabilisation des corps gras contenus dans les différents laits, crèmes, onguents, produits solaires notamment huiles solaires etc...

Commes huiles essentielles convenant plus particulièrement au domaine de la parfumerie et de la cosmétique, on peut citer l'essence de bergamote, l'essence de lavande, l'essence de néroli, l'essence de rose, l'essence de santal, l'essence de vétiver etc ... On peut faire appel également, à toutes les essences connues et décrites dans la littérature (cf. ENCYCLOPEDIA CHEMICAL TECHNOLOGY - KIRK OTHMER - Volume 14 OILS, ESSENTIAL).

En ce qui concerne la nature des corps gras intervenant dans les différents produits de beauté et d'hygiène, elle est très variée et l'on mentionnera seulement quelques exemples qui pourront être complétés en se reportant à l'encyclopédie ENCYCLOPEDIA CHEMICAL TECHNOLOGY - KIRK OTHMER - Volume 19 COSMETICS et qui sont : l'huile d'amandes douces ou amères, la cire d'abeilles, la lanoline, des huiles minérales, la paraffine, le blanc de baleine, l'huile de ricin, la cérésine, le beurre de cacao, l'huile d'olive, l'acide oléique, l'huile d'arachide, l'huile de sésame, l'acide stéarique etc ...

Les émulsions stabilisées des huiles essentielles et des différents corps gras sont préparées selon les mêmes techniques décrites que celles destinées à l'industrie alimentaire.

Les préparations de sucroglycérides sous forme fluide selon l'invention peuvent être utilisées dans l'industrie de la cuisson des céréales.

On sait que la mise en oeuvre de sucroglycérides au cours de la fabrication d'une pâte, terme pris dans un sens très large de mélange de farine et d'eau, permet des améliorations d'une part, au niveau de la préparation de la pâte : temps de liaison de la pâte diminuée, temps de pétrissage plus court, incorporation de gras facilitée et travail de la pâte plus facile et d'autre part, au niveau des caractères de la pâte : texture plus fine du produit obtenu (cf. brevet français n° 1 483 716 - PV n° 65 152).

Un domaine d'application privilégié des préparations de l'invention est celui de la biscuiterie, biscotterie ou pâtisserie et plus particulièrement :

- biscuiterie sèche ; biscuits sucrés de type classique, petits beurre, galettes, casse-croûte, sablés, biscuits·salés pour apéritifs, crackers.

- pâtisserie industrielle : boudoirs, champagne, langues de chat, biscuits à la cuillère, pain de gênes, génoise, madeleines, quatre-quarts, cakes, pâtisserie aux amandes, petits fours.

- biscotterie.

- pâtes feuilletées levées (croissants) et non levées (vol-auvent).

- pâtes à foncer (pâtes sablées, pâtes brisées sucrées ou non sucrées, type traiteur).

Les éléments fondamentaux présents dans les mélanges destinés aux industries précitées sont les protéines (gluten) et l'amidon qui sont les plus souvent apportés par la farine de froment. Pour la préparation des divers types de biscuits et gâteaux, on ajoute à la farine des ingrédients tels que saccharose, sel, oeufs, lait, corps gras, éventuellement levures chimiques (bicarbonate de sodium ou autres levures artificielles) ou levures biologiques et farines de céréales diverses etc...

L'incorporation des préparations des sucroglycérides selon l'invention est réalisée au cours de la fabrication en fonction du produit souhaité et est conduite selon les techniques classiques du domaine considéré (cf. J.L. KIGER et J.G. KIGER - Techniques Modernes de la Biscuiterie Pâtisserie-Boulangerie industrielles et artisanales et des produits de régime, DUNOD, Paris, 1968, Tome 2, pages 231 et suivantes). D'une manière préférentielle, elles sont introduites dans la phase finale de la préparation de la pâte.

Les quantités de préparations de l'invention mises en jeu et exprimées en poids de sucroglycérides représentent de 0,3 à 2 % du poids de farine utilisée.

Avant de concrétiser par des exemples, les possiblités d'utilisation des préparations de l'invention, on donne des exemples sans aucun caractère limitatif illustrant les préparations de l'invention et leur procédé d'obtention (exemples 1 à 8).

A titre de comparaison, on réalise un certain nombre d'essais mettant en évidence les paramètres critiques de leur procédé de fabrication.

- influence de l'agitation dans l'étape du mélange sucroglycérides et huile (essai A) et, en plus, dans la phase de refroidissement (essai B)

- influence de la température de refroidissement du mélange (essai C)

- influence de l'agitation finale de la préparation de l'invention (essai D).

Dans tous les exemples et essais, les pourcentages sont exprimés en poids.


EXEMPLE 1

On décrit ci-après le procédé d'obtention d'une préparation de sucroglycérides sous forme fluide à partir des sucroglycérides et de l'huile de colza dont les caractéristiques sont les suivantes :

Les sucroglycérides utilisés à titre de matières premières sont des sucroglycérides d'huile de palme commercialisés par la Société RHONE-POULENC sous la dénomination commerciale CELYNOL MSPO 11 dont les spécifications sont les suivantes :

- Saccharose combiné ..................... $19 \pm 2$ %
- Saccharose libre ....................... $\leqslant 1,5$ %
- Indice d'acide en mg de KOH/g........... $\leqslant 8$
- Indice de saponification en mg de KOH/g.. $150 \pm 10$
- Densité à 66°C ......................... 0,97
- Viscosité cinématique en m$^2$/s à 98,9°C ..$(210 \pm 80)10^{-6}$
- Zone de fusion ......................... 50 à 60°C

L'huile de colza est une huile de colza raffinée caractérisée de la manière qui suit :

- composition moyenne en acides gras exprimée en pourcentages du poids total des acides gras
  . acide palmitique ........................ ·2.9 - 3.8
  . acide stéarique ........................ 1.2 - 5.7
  . acide oléique ........................ 59.4

. acide linoléique ..................... 11.9 - 17.9

. acide linolénique ..................... 5.9 -  9.4

. acide arachidique ..................... 0.7 -  1.2

. acide erucique ..................... 0.50

- densité moyenne ............... 0.910 - 0.916 à 20°C

- point de solidification ....................... - 6°C

- point de fusion ......................... 18°C - 22°C

- indice de réfraction ......................... 1.4745

- solubilité dans l'alcool à 15°C .......... 20 g/100 cm$^3$

- indice de saponification en mg de KOH/g... 175 - 179

- indice d'iode en g de I$_2$/100 g............ 97.7 - 106

Dans un fondoir de 1 000 litres muni d'une agitation à pâles montées sur un arbre tournant à 60 tours/minute et d'une double enveloppe, on introduit 800 kg de sucroglycérides.

On fait fondre les sucroglycérides en augmentant la température par injection de vapeur soufflante dans la double enveloppe. Il est préférable que la température des sucroglycérides fondus ne dépasse pas 75°C.

Dans une cuve de préparation de 3 000 litres munie d'une agitation à cadre montée sur un arbre tournant à 20 tours/minute et d'une double enveloppe, on introduit les 800 kg de sucroglycérides fondus à une température de 70 - 75°C.

On n'y ajoute sous agitation en continu pendant une durée de 30 minutes par un système de pompe centrifuge, 1 200 kg d'huile de colza qui est à température ambiante (de 16 à 22°C). La température du mélange tombe alors à 50 - 55°C en fin d'addition de l'huile.

On maintient l'agitation 5 minutes à une vitesse de 20 tours/minute puis elle est interrompue.

On procède au refroidissement du mélange en abaissant sa température par circulation d'une eau refroidie de 12 à 15°C dans une double enveloppe.

Il y a lieu quelquefois d'agiter le mélange par intermittence afin d'égaliser la température dans la masse du mélange : 30 secondes toutes les demi-heures.

Le refroidissement est poursuivi jusqu'à ce que la température du mélange atteigne 18 à 20°C ; il dure environ 5 heures.

A 18 - 20°C, le mélange est agité pendant 10 minutes à 20 tours/minute sous refroidissement.

On obtient une préparation ayant l'aspect d'une huile épaisse et qui a les propriétés suivantes :

- viscosité : la viscosité a été mesurée à différentes températures au viscosimètre BROOKFIELD, modèle RVT à la vitesse de 5 tours/minute et avec l'aiguille n° 6.

Les résultats obtenus sont les suivants :

(Tableau I)

| Température (°C) | + 6° | + 10° | + 14° | + 16° | + 18° |
|---|---|---|---|---|---|
| Viscosité (mPa.s) | 26 000 | 20 000 | 12 000 | 9 000 | 7 000 |

On remarque donc une baisse importante de la viscosité entre 6 et 18°C et que la préparation de l'invention peut être aisément transvasée dès 10°C.

- densité : la densité mesurée à 20°C est de 0,95
- concentration en matière active : 40 %
- stabilité mécanique : entre 5 et 50°C, passage de l'état pâteux à l'état liquide et inversement sans séparation de phases
- stabilité chimique : bonne
- stabilité bactérienne : risque nul
- dispersabilité dans l'eau : le test suivant met en évidence les bonnes propriétés de dispersabilité dans l'eau des sucroglycérides de l'invention comparées à celles des sucroglycérides seuls et des sucroglycérides sur support sucré.

Les caractéristiques des produits comparés sont les suivantes :
. sucroglycérides d'huile de palme commercialisés sous la marque CELYNOL MSPO 11
. sucroglycérides sur support sucré mis sur le marché sous la dénomination CELYNOL F1 : ils se présentent sous forme d'une pâte épaisse de coloration jaune ayant la composition suivante :

- sucroglycérides d'huile de palme ......... 40 %

- support sucré ............................ 30 %

- eau ...................................... 30 %

(le support sucré est un sucre interverti obtenu par hydrolyse du saccharose et qui se présente sous l'aspect d'une pâte blanche très oncteuse dont l'analyse est la suivante :

     - brix réfractométrique ..................... 79,90,

     - matières sèches .......................... 81,66 %

       dont 39,48 % de dextrose, 39,48 % de lévulose et

       2,70 % de saccharose résiduaire)

     - densité ................................... 1,411,

     - pH ....................................... 5,5).

Le test de dispersabilité consiste à disperser 20 g de l'échantillon à tester dans 500 cm$^3$ d'eau distillée à 20°C à l'aide d'une agitation constituée par deux pâles rectangulaires de 17 mm de hauteur, décrivant un cercle de 76 mm de diamètre et disposées verticalement sur un axe tournant à la vitesse constante de 200 tours/minute.

Les résultats sont les suivants :

. la répartition de l'échantillon de sucroglycérides de l'invention s'est effectuée dans l'eau en 30 secondes : on obtient un dispersion homogène

. il a fallu 1 heure pour disperser 12 g de sucroglycérides sur support sucré : 40 % de la masse initiale de l'échantillon n'ont pas été dispersés

. les sucroglycérides seuls ne sont absolument pas dispersables.

- couleur : jaune clair

- odeur : très faible odeur d'huile

- saveur : légèrement amère

- conforme à la législation alimentaire.


ESSAIS A et B

On réalise la fabrication d'une préparation obtenue à partir de sucroglycérides d'huile de palme et d'huile de colza d'une manière identique à l'exemple 1 sauf en ce qui concerne les conditions d'agitation.

Dès l'introduction de l'huile de colza dans les sucroglycérides fondus, on maintient une agitation continue et énergique d'environ 50 tours/minute, jusqu'à ce que le mélange soit homogène : la température est alors comprise entre 50 et 55°C.

Dans ces conditions, l'agitation est trop violente et provoque rapidement la formation de mousses en surface qui donnent au refroidissement réalisé conformément à l'exemple 1, des nodules d'apparence différente du milieu et qui ne se mélangent plus au reste de la préparation.

Si l'agitation forte est également maintenue pendant la phase de refroidissement, on incorpore dans la préparation, une grande quantité d'air et transforme celle-ci en mousse.

Ces essais montrent, que l'agitation au cours de fabrication des préparations doit être douce et limitée.

ESSAI C

Dans cet essai, on met en évidence l'influence d'une température minimum de refroidissement sur l'obtention d'une préparation fluide.

On a simulé la phase de refroidissement par le test suivant :
- dans un bécher de 500 cm$^3$, on introduit 300 g du mélange de sucroglycérides et d'huile de colza à la température de 50°C et obtenus dans les mêmes conditions que celles décrites dans l'exemple 1
- on plonge l'aiguille d'un viscosimètre BROOKFIELD, modèle RVT et un thermomètre et l'on suit l'évolution de la viscosité en fonction de l'abaissement de la température.

Les viscosités obtenues sont les suivantes :

(Tableau II)

| Température (°C) | Viscosité (mPa.s) |
|---|---|
| 50 à 35 | 100 |
| 34 | 100 |
| 33 | 140 |
| 32 | 150 |
| 31 | 160 |
| 30 | 180 |
| 29 | 200 |
| 28 | 500 |
| 27 | 700 |
| 26 | 800 |
| 25 | 1 100 |
| 24 | 3 000 |
| 23 | 6 000 |
| 22 | 10 000 |
| 21 | non mesurable avec le viscosimètre BROOKFIELD, modèle RVT |

On remarque que pour obtenir une préparation fluide de manière irréversible, il est nécessaire de détruire l'édifice cristallin après refroidissement à une température inférieure à 22°C et de préférence entre 15 et 20°C.

Si l'on arrête le refroidissement a une température supérieure à 22°C et si l'on agite le mélange, on risque d'obtenir une préparation susceptible de se rigidifier au cours d'un abaissement de température.

0091331

26

ESSAI D

Dans cet essai, on reproduit l'exemple 1 à la différence près que l'on supprime toute agitation pendant la phase de refroidissement jusqu'à la température de 15°C.

On obtient une masse gélifiée, non pompable ayant une viscosité de 150 000 à 200 000 mPa.s.

Si l'on agite ensuite, le mélange, ce dernier devient fluide et le reste ensuite, même si la température est abaissée jusqu'à 10°C.

EXEMPLES 2 à 5

On réalise la fabrication de nouvelles préparations en reproduisant l'exemple 1 à la différence près que l'on remplace l'huile de colza par les huiles suivantes :

- exemple 2   : huile de tournesol
                (point de solidification = -17°C)
- exemple 3   : huile de soja
                (point de solidification  = -16°C)
- exemple 4   : huile d'arachide
                (point de solidification = +3°C)
- exemple 5   : huile de maïs
                (point de solidification = -20°C)

On obtient des préparations semblables à celles mettant en jeu l'huile de colza qui se différencient seulement par leurs viscosités mesurées à 20°C dans les mêmes conditions que décrites précédemment et qui sont les suivantes :

(Tableau III)

| EXEMPLES | NATURE DE L'HUILE | VISCOSITE (mPa.s) |
|----------|-------------------|-------------------|
| 2 | Huile de tournesol | 2 500 |
| 3 | Huile de soja | 5 000 |
| 4 | Huile d'arachide | 12 500 |
| 5 | Huile de maïs | 3 500 |

On note donc que quelle que soit l'huile utilisée, on reste dans une zone de viscosité permettant le transvasement des préparations par gravité ou pompage.

EXEMPLE 6

On décrit ci-après le procédé d'obtention d'une préparation de sucroglycérides sous forme fluide à partir de sucroglycérides tels que définis dans l'exemple 1 et de l'huile de soja dont les caractéristiques sont les suivantes :

- composition moyenne en acides gras principaux exprimée en pourcentages du poids total des acides gras :
  - . acide palmitique.................. 9,9 - 1,21
  - . acide stéarique.................. 1,3 - 4,8
  - . acide oléique................... 22,5 - 31,2
  - . acide linoléique................ 48,9 - 54,7
  - . acide linolénique...............∴. 5,2 - 8,5
- densité......0,922 à 20°C
- point de solidification..... −8 à −18°C
- indice de réfraction à 40°C............... 1,465 - 1,469
- indice de saponification en mg de KOH/g... 192,5
- indice d'iode en g de $I_2$/100 g............ 121,30

Dans un fondoir de 1000 litres muni d'une agitation à pâles montées sur un arbre tournant à 60 tours/minutes et d'une double-enveloppe, on introduit 560 kg de sucroglycérides.

On fait fondre à 80°C les sucroglycérides en augmentant la température par injection de vapeur soufflante dans la double-enveloppe.

Dans une cuve de préparation de 3000 litres munie d'une agitation à cadre montée sur un arbre tournant à 30 tours/minute et d'une double enveloppe, on introduit 840 kg d'huile de soja à 24°C.

On y ajoute sous agitation en continu pendant une durée de 15 minutes, par un système de pompe centrifuge, les 560 kg de sucroglycérides fondus à une température de 75°C. La température d'équilibre dans la cuve de préparation à la fin de l'addition des sucroglycérides est de 45°C.

On maintient l'agitation 5 minutes à une vitesse de 20 tours/minute puis elle est interrompue.

On procède au refroidissement du mélange en abaissant sa température par circulation d'eau refroidie à 3°C dans une double-enveloppe.

Il y a lieu quelquefois d'agiter le mélange par intermittence afin d'égaliser la température de la masse du mélange : 30 secondes toutes les demi-heures.

Le refroidissement est poursuivi jusqu'à ce que la température du mélange atteigne 8°C : il dure 6 heures.

A la fin de l'opération, le mélange est agité pendant 5 minutes à 20 tours/minute sous refroidissement.

Le conditionnement se fait en fûts par gravité.

On obtient une préparation ayant l'aspect d'une huile épaisse ayant une concentration en matière active de 40 % et une viscosité de 5 000 mPa.s.

EXEMPLE 7 -

On donne, ci-après, le procédé d'obtention d'une préparation améliorée de sucroglycérides sous forme fluide comprenant des sucroglycérides d'huile de palme, de la lécithine et de l'huile de soja.

Les sucroglycérides sont définis dans l'exemple 1.

La lécithine employée est de la lécithine de soja qui est un produit brun-clair semi-fluide commercialisé par la Société LECITHOS et formé par un mélange de plusieurs phosphatides à prédominance phosphatidyl-choline.

L'huile de soja est caractérisée dans l'exemple 6.

Dans un fondoir de 1 000 litres muni d'une agitation à pâles montées sur un arbre tournant à 60 tours/minute et d'une double enveloppe, on introduit 640 kg de sucroglycérides.

On fait fondre les sucroglycérides en augmentant la température par injection de vapeur soufflante dans la double enveloppe. Il est préférable que la température des sucroglycérides fondus ne dépasse pas 75°C.

Dans les sucroglycérides fondus, on introduit sous agitation, par l'intermédiaire d'une pompe centrifuge, 160 kg de lécithine de soja.

Dans une cuve de préparation de 3 000 litres munie d'une agitation à cadre montée sur un arbre tournant à 20 tours/minute et d'une double enveloppe, on introduit les 800 kg de mélange constitué par les sucroglycérides et la lécithine de soja qui est à une température de 70-75°C.

On n'y ajoute sous agitation en continu pendant une durée de 30 minutes par un système de pompe centrifuge, 1 200 kg d'huile de soja qui est à température ambiante (de 16 à 22°C). La température du mélange tombe alors à 50 - 55°C en fin d'addition de l'huile.

On maintient l'agitation 5 minutes à une vitesse de 20 tours/minute puis elle est interrompue.

On procède au refroidissement du mélange en abaissant sa température par circulation d'une eau refroidie de 12 à 15°C dans une double enveloppe.

Il y a lieu quelquefois d'agiter le mélange par intermittence afin d'égaliser la température dans la masse du mélange : 30 secondes toutes les demi-heures.

Le refroidissement est poursuivi jusqu'à ce que la température du mélange atteigne 18 à 20°C ; il dure environ 5 heures.

A 18 - 20°C, le mélange est agité pendant 10 minutes à 20 tours/minute sous refroidissement.

On obtient une préparation ayant l'aspect d'une huile épaisse qui a les propriétés suivantes :

  - composition :

    . sucroglycérides d'huile de palme........... 32 %

    . lécithine de soja......................... 8 %

    . huile de soja.............................. 60 %

  - viscosité : la viscosité a été mesurée à différentes températures au viscosimètre BROOKFIELD, modèle RVT à la vitesse de 5 tours/minute et avec l'aiguille n° 6 à 10 et 20°C, l'aiguille n° 5 à 30 et 40°C, l'aiguille n° 4 à 50°C.

Les résultats obtenus sont les suivants :

Tableau IV

| | | | | | |
|---|---|---|---|---|---|
| Température (°C) | + 10° | + 20° | + 30° | + 40° | + 50° |
| Viscosité (mPa.s) | 12 000 | 3 500 | 1 400 | 200 | 100 |

On remarque une baisse importante de la viscosité entre 10° et 20°C.

Les résultats obtenus sont comparés à ceux des sucroglycérides seuls (tableau V) et d'une préparation de sucroglycérides sous forme fluide obtenue contenant 40 % de sucroglycérides d'huile de palme et 60 % d'huile de soja (Tableau VI).

Les viscosités sont mesurées dans les mêmes conditions que décrites précédemment sauf pour les sucroglycérides seuls où l'aiguille utilisée à 40 et 50°C est l'aiguille n° 6.

Tableau V

| | | | | | |
|---|---|---|---|---|---|
| Température (°C) | + 10° | + 20° | + 30° | + 40 | + 50° |
| Viscosité (mPa.s) | non mesurable | non mesurable | non mesurable | 36 000 | 6 000 |

Tableau VI

| | | | | | |
|---|---|---|---|---|---|
| Température (°C) | + 10° | + 20° | + 30° | + 40° | + 50° |
| Viscosité (mPa.s) | 20 000 | 5 000 | 3 500 | 400 | 160 |

L'analyse comparative des Tableaux IV et VI montre une amélioration de la fluidité des préparations avec lécithine pour une même teneur en huile.

- stabilité mécanique        : bonne
- stabilité chimique        : bonne
- stabilité bactérienne        : risque nul
- dispersabilité dans l'eau    : on note une meilleure
  dispersabilité des préparations améliorées par rapport aux
  sucroglycérides seuls ou aux préparations sans lécithine
- couleur            : jaune-brun
- odeur            : très faible odeur d'huile
- saveur            : légèrement amère
- conforme à la législation alimentaire

EXEMPLE 8 -

Cet exemple illustre une préparation de sucroglycérides sous forme fluide contenant des sucroglycérides de suif, de la lécithine de soja et de l'huile de colza.

Les sucroglycérides de suif utilisés à titre de matières premières sont commercialisés par la Société RHONE-POULENC sous la dénomination commerciale CELYNOL MST 11 dont les spécifications sont les suivantes :

- Saccharose combiné........................... $19 \pm 2$ %
- Saccharose libre............................. $\leqslant 1,5$ %
- Indice d'acide en mg de KOH/g................ $\leqslant 8$
- Indice de saponification en mg de KOH/g...... $150 \pm 10$
- Viscosité cinématique en $m^2/s$ à 100°C.......$(220 \pm 80)\ 10^{-6}$
- Zone de fusion............................... 50 à 60°C

L'huile de colza est celle qui a été caractérisée dans l'exemple 1.

La préparation faite conformément au mode opératoire de l'exemple 7 a la composition suivante :

. sucroglycérides de suif................... 42 %
. lécithine de soja........................ 18 %
. huile de colza........................... 40 %

Les mesures de viscosité réalisées au viscosimètre BROOKFIELD modèle RVT à la vitesse de 5 tours/minute et avec l'aiguille n° 6 à 10 et 20°C, l'aiguille n° 5 à 30°C, l'aiguille n° 4 à 40°C et l'aiguille n° 3 à 50°C sont les suivantes :

Tableau VII

| : | : Température (°C) | : + 10° | : + 20° | : + 30° | : + 40° | : + 50° | : |
|---|---|---|---|---|---|---|---|
| : Viscosité (mPa.s) | : 9 000 | : 2 500 | : 1 400 | : 200 | : 100 | : |

Les résultats obtenus sont comparés à ceux des sucroglycérides seuls (Tableau VIII) et d'une préparation contenant 60 % de sucroglycérides de suif et 40 % d'huile de colza (Tableau IX).

Les viscosités sont mesurées dans le cas des sucroglycérides de suif, avec l'aiguille n° 6 à 40° et 50°C et dans le cas de la préparation sans lécithine, avec l'aiguille n° 6 à 30 et 40°C et l'aiguille n° 5 à 50°C.

Tableau VIII

| : | : Température (°C) | : + 10° | : + 20° | : + 30° | : + 40° | : + 50° | : |
|---|---|---|---|---|---|---|---|
| : Viscosité (mPa.s) | : non mesu-rable | : non mesu-rable | : non mesu-rable | : 23 000 | : 5000 | : |

Tableau IX

| : | : Température (°C) | : + 10° | : + 20° | : + 30° | : + 40° | : + 50° | : |
|---|---|---|---|---|---|---|---|
| : Viscosité (mPa.s) | : non mesu-rable | : non mesu-rable | : 16 000 | : 2 000 | : 400 | : |

L'étude comparative des tableaux VIII et IX montre que l'addition de l'huile, aux sucroglycérides de suif permet d'abaisser, d'une manière significative, leur viscosité mais que le résultat obtenu n'est pas encore satisfaisant lorsque l'on travaille à température ambiante.

On note que l'addition de la lécithine entraîne une diminution de la viscosité dans une zone qui permet le transvasement des préparations par gravité ou pompage.

Suivent des exemples d'application des préparations de l'invention à la confection d'émulsions stables d'huiles essentielles, d'une composition de lait réengraissé pour veaux et dans l'industrie de cuisson des céréales.

Les exemples 9 et 10 illustrent l'utilisation des préparations de sucroglycérides de l'invention pour l'obtention d'émulsions stables d'huiles essentielles.

EXEMPLE 9

Dans cet exemple, on prépare une émulsion d'essence d'orange ayant la composition suivante :

- essence d'orange douce Afrique ..................... 10 g
- sucroglycérides de l'invention obtenus selon
  l'exemple de préparation n° 1 ....................... 10 g
- eau ............................................... 80 g

Le mode de préparation de l'émulsion est donné ci-après :

- dans un bécher de 200 cm$^3$ surmonté d'un système d'agitation à turbine tournant à 5 000 tours/minute et équipé d'un thermomètre et d'un dispositif de chauffage, on introduit 80 g d'eau de ville ;

- on porte cette eau à une température de 70 - 75°C ;

- à cette température, on ajoute sous agitation et progressivement 10 g d'essence d'orange ;

- on ajoute progressivement, toujours sous agitation les sucroglycérides de l'invention lorsque l'essence d'orange est complètement dispersée ce qui est obtenu au bout d'une minute.

On obtient une émulsion d'essence d'orange, fine et stable, ayant l'aspect d'un lait bleuté.

EXEMPLE 10

Suit un exemple de préparation d'une émulsion d'anis ayant la composition donnée ci-après :

- essence d'anis ...................................... 3 g
- sucroglycérides de l'invention obtenus selon
  l'exemple de préparation n° 1....................... 5 g
- eau ............................................... 50 g

La préparation de l'émulsion est conduite selon la technique opératoire de l'exemple 9.

On obtient une émulsion stable d'essence d'anis dont les globules ont un diamètre déterminé au microscope de 0,1 à 0,5 µ. On peut parfaire l'émulsion par passage dans un homogénéiseur à pression MANTON - GAULIN. Cette opération consiste à forcer sous pression de 200 bars, l'émulsion à travers des filières, des pas de vis afin de réduire la dimension des globules entre 0,05 et 0,01 µ.

Les émulsions obtenues présentent une grande stabilité au stockage : au bout d'un an, elles n'ont pas évolué.

Dans l'exemple 11 qui suit, on exemplifie la misé en oeuvre de la préparation de l'invention dans un lait de réengraissement pour veaux.

On réalise à titre comparatif, un essai dans lequel la préparation de l'invention est remplacée par une composition témoin ne contenant pas d'huile (essai E).

EXEMPLE 11
ESSAI E

Pour réaliser ces essais, on dispose
- d'une poudre de lait réengraissé contenant 40 % de suif
- d'une poudre de lait maigre
- du produit à tester.

Pour les besoins de l'expérience, le produit à tester est mis sur support poudre de lait. Pour ce faire, on pulvérise 30 g de sucroglycérides décrits dans l'exemple 8 sur 70 g de poudre de lait.

Dans le cas de l'essai comparatif, on fond à 75°C, 70 g de sucroglycérides de suif dans lesquels on introduit 30 g de lécithine de soja puis 30 g du mélange fondu sont pulvérisés sur 70 g de poudre de lait maigre.

On prépare ensuite la poudre de lait réengraissé, en mélangeant dans un mélangeur à hélice à 6 pales (type pale défloculeuse de RAYNERI), 36,5 g de poudre de lait maigre, 37,5 g de poudre de lait contenant 40 % de suif et 1 g de sucroglycérides de l'invention mis sur support dans l'exemple 11 ou 1 g de la composition-témoin précitée mise également sur support dans l'essai E.

On reconstitue ensuite le lait par addition de 450 cm$^3$ d'eau chaude dont la température varie selon le test.

On soumet les différents laits obtenus à une série de tests afin de voir si la présence de l'huile ne gêne pas au niveau des propriétés du lait reconstitué.

Les paramètres étudiés sont les suivants :

- formation des mousses

- qualité de l'émulsion : lorsque la matière grasse s'émulsionne mal, elle a tendance à former des agglomérats qui sous l'effet de l'agitation vont conduire au phénomène de barattage.

- stabilité de l'émulsion : elle est évaluée par la remontée de la matière grasse à la surface du lait.

A - TEST DE FORMATION DE MOUSSES -

Le test permet
de chiffrer la quantité de mousses formées,
de noter l'aspect des mousses (fines et serrées, où très aérées),
de connaître la stabilité dans le temps des mousses formées.

1°) Principe du test -

Un volume donné de lait reconstitué est mis sous agitation importante pendant un temps fixé.

On mesure la hauteur de mousses formées et l'évolution de cette hauteur dans le temps.

2°) Appareillage -

. Agitateur Ultra - Turrax TP 18 - 2 K, réglé sur la vitesse maximale (vitesse du rotor environ 20 000 tours/minute),

. Béchers cylindriques de 75 mm de diamètre et 185 mm de hauteur ou, à défaut, béchers de 800 cm³, forme haute,

. Eprouvettes graduées de 500 cm3,

. Balance,

. Chronomètres,

. Réglettes graduées en mm que l'on peut fixer à l'extérieur du bécher.

3°) Mode opératoire —

Dans le bécher, on verse 450 cm³ d'eau de ville à 45°C. On ajoute 75 g de poudre de lait à tester; on la mouille très rapidement en agitant quelques secondes avec une baguette de verre. On agite aussitôt à 20 000 tours/minute pendant 2 minutes.

On mesure avec la réglette graduée, la hauteur de mousses formées après : 3 - 5 - 10 - 15 - 20 - 25 - 30 et 60 minutes.

4°) Expression des résultats —

Les résultats sont exprimés en mm de hauteur de mousses et les valeurs sont comparées entre elles.

5°) Résultats —

Les résultats obtenus sont consignés dans le tableau X.

TABLEAU X

|  | Exemple 11 | Essai E |
|---|---|---|
| Produits à tester | Sucroglycérides de l'invention | Sucroglycérides et lécithine |
| Temps en minute | Hauteur de mousses en mm | |
| 3 | 17 | 21 |
| 5 | 16 | 20 |
| 10 | 7 | 15 |
| 15 | 3 | 9 |
| 20 | 0 | 6 |
| 25 | — | 3 |
| 30 | — | 1 |
| 60 | — | 0 |

Au niveau de la formation des mousses, on note une diminution favorable des mousses avec la préparation de l'invention.

B - TEST DE BARATTAGE

Il permet de mettre en évidence le phénomène de barattage qui se traduit par l'apparition de grumeaux de matière grasse, plus ou moins gros, en surface et sur la paroi du bécher juste au-dessus du liquide.

1°) Principe du test -

Un volume donné de lait est reconstitué sous agitation ayant un effet de cisaillement important.

Après disparition de la mousse, la matières grasse qui a baratté apparaît en surface.

2°) Appareillage -

. Moteur tournant à 1 500 tours/minute entraînant un arbre muni d'une pale (type pale défloculeuse de RAYNERI),

. Béchers de 2 litres,

. Béchers de 600 cm$^3$ forme basse,

. Eprouvettes graduées de 500 cm$^3$,

. Balance,

. Chronomètres.

3°) Mode opératoire -

Dans le bécher de 2 litres, on verse 450 cm$^3$ d'eau de ville à 60°C. On ajoute 75 g de poudre de lait à tester, on agite quelques secondes à la baguette de verre pour la mouiller. On agite aussitôt à 1500 tours/minute pendant 15 minutes.

On verse le lait doucement dans le bécher de 600 cm$^3$ en observant si des blocs de matière grasse apparaissent en surface ou dans la mousse.

Lorsque la mousse a disparu, on observe la surface et la paroi juste au-dessus du liquide ; s'il y a barattage il apparait sous forme de grains plus ou moins gros.

4°) Expression des résultats -

Les résultats sont exprimés de façon qualitative et permettent un classement entre différents produits.

5°) Résultats -

Les laits reconstitués contenant la préparation de l'invention ou la composition-témoin ne donnent pas de barattage.

C - <u>TEST DE LA REMONTEE DE LA MATIERE GRASSE</u> -

La méthode pour la détermination de la teneur en matière grasse d'un lait, décrite dans la norme NF - V 04 - 210 a été reprise pour la détermination de la matière grasse totale du lait et a été adaptée à la mesure de la remontée de matière grasse dans le lait reconstitué laissé au repos.

1°) Principe -

L'analyse acido-butyrométrique consiste en une hydrolyse par l'acide sulfurique, qui dissout les protéines du lait. La séparation de la matière grasse est facilitée par l'ajoût d'une faible quantité d'alcool isoamylique et par une centrifugation.

Par lecture directe sur l'échelle du butyromètre, on a la teneur en matière grasse, en grammes pour 100 cm$^3$ de lait ou en grammes pour 100 grammes de lait, selon le volume de lait prélevé pour l'analyse.

Pour déterminer la quantité de matière grasse remontée dans un lait en fonction du temps, on dose après repos, au temps t, la quantité de matière grasse restant dans la phase inférieure (représentée par les 3/4 du volume total) et, connaissant la matière

grasse totale, on peut calculer la quantité de matière grasse contenue au temps t dans la phase supérieure et l'enrichissement % de cette phase.

2°) Appareillage —

. Moteur tournant à 1300 tours/minute $\pm$ 50, entraînant un arbre terminé par une hélice à 6 pales (type pale éventail de RAYNERI), de 45 mm de diamètre,

. Ampoules à brome de 250 cm3, dont la partie supérieure est sciée pour pouvoir y introduire l'agitateur,

. Eprouvettes de 100 $cm^3$, bouchées émeri,

. Pipettes à lait de 11 cm3, à 1 trait (NF – B 35 523) (avec ce volume de lait, l'échelle du butyromètre donnera directement le résultat en grammes de matière grasse pour 100 $cm^3$ de lait),

. Pipettes de 1 cm3 et de 10 cm3 à 1 trait,

. Butyromètres à lait, échelle 0/6 %, munis de bouchons appropriés,

. Centrifugeuse pour butyromètre (vitesse de rotation : 1100 à 1300 tours/minute selon les modèles),

. Bain d'eau thermostaté à 45°C et thermomètre approprié,

. Bain d'eau thermostaté à environ 75°C et thermomètre approprié,

. Bain d'eau thermostaté à 65°C $\pm$ 2 et thermomètre approprié,

. Balance analytique.

3°) Réactifs —

. Acide sulfurique pour analyse du lait d = 1,820 $\pm$ 0,005,

. Alcool isoamylique pur pour analyse du lait.

4°) Mode opératoire —

a – Reconstitution du lait

. On règle la pale d'agitation au fond de l'ampoule à brome, on verse 92 $cm^3$ d'eau de ville à 52 $\pm$ 1°C, et 11 g de poudre de lait à tester, pesés exactement à la balance de précision,

. On agite une minute à 1300 tours/minute en aidant, si nécessaire, la mise en solution de la poudre collée sur les parois du récipient ou sur la tige de l'agitateur,

. On prélève aussitôt 11 cm$^3$ de lait reconstitué, pour le dosage de la matière grasse totale, au temps zéro, Pour le dosage à différents temps de repos, on fait une reconstitution de lait pour chacun de ces temps (5 minutes — 15 minutes et 30 minutes) et on porte les ampoules au bain-marie à 45°C pendant le repos,

. On soutire 75 cm3 de lait, on agite pour homogénéiser, puis on prélève aussitôt 11 cm$^3$ pour le dosage de la matière grasse.

b - Dosage de la matière grasse

. On mesure 10 cm3 d'acide sulfurique et les introduit dans le butyromètre, en opérant de façon que l'acide n'en mouille pas le col et n'entraîne pas d'air,

. On agite la fraction de lait à analyser pour l'homogénéiser, on prélève immédiatement 11 cm$^3$ avec la pipette à lait et on les verse dans le butyromètre, de façon qu'ils forment une couche au-dessus de l'acide et sans mouiller le col,

. On mesure 1 cm$^3$ d'alcool isoamylique et on l'introduit dans le butyromètre sans mouiller le col et sans mélanger les liquides,

. On bouche bien le butyromètre avec un bouchon conique en caoutchouc,

. On agite lentement jusqu'à ce que les protéines du lait soient entièrement dissoutes, c'est-à-dire qu'il n'y ait plus de particules blanches,

. On place ensuite le butyromètre, bouchon en bas, dans le bain-marie à 75°C pendant 10 minutes,

. En agissant sur le bouchon, on amène la colonne de matière grasse dans la zone de lecture.

. On place le butyromètre, bouchon en bas, dans le bain-marie à 65°C pendant quelques minutes pour la mise en équilibre de température avant la lecture.

5°) Expression des résultats

Par lecture directe, on a la teneur en matière grasse de la phase considérée exprimée en grammes pour 100 cm$^3$ de lait.

Si n est la lecture du butyromètre au temps zéro et n' la lecture du butyromètre au temps t, on calcule par différence l'enrichissement en pourcentage de matière grasse de la phase supérieure selon la formule

$$\frac{3(n - n')}{n} \times 100$$

6°) Résultats –

Les résultats obtenus avec la préparation de l'invention et la composition-témoin sont consignés dans le tableau XI.

Tableau XI

| Produits à tester | Exemple 11 | Essai E |
|---|---|---|
| | Sucroglycérides de l'invention | Sucroglycérides et lécithine |
| Temps en minutes | Enrichissement en % de la phase supérieure | |
| 5 | 0 | 0 |
| 15 | 6,25 | 6,25 |
| 30 | 12,50 | 12,50 |

On remarque donc que la stabilité des laits obtenus est comparable.

La présence d'huile n'affecte pas les propriétés émulsifiantes des sucroglycérides.

En conclusion, la mise des sucroglycérides sous la forme décrite par la présente invention apporte d'énormes avantages au niveau de leur mise en oeuvre tout en n'entraînant aucun inconvénient au niveau de l'application voire-même amenant des propriétés meilleures notamment en ce qui concerne la formation des mousses.

L'introduction des préparations de l'invention au cours de la fabrication des pâtes modifient leurs propriétés rhéologiques et influencent leur comportement au cours de la chaîne de fabrication ainsi que les caractéristiques des produits finis obtenus.

L'exemple suivant (exemple 12) met en évidence les effets apportés par la préparation au moyen des tests classiques de détermination de caractéristiques de farines ou de pâtes. Le paramètre étudié est l'action sur le gluten mesurée avec le farinographe BRABENDER.

On réalise à titre comparatif, un essai–témoin sans additif (essai F) et des essais comparatifs contenant séparément les constituants de la préparation de l'invention (essais G et H).

Enfin, on compare l'effet des sucroglycérides de l'invention à celui obtenu par les sucroglycérides déposés sur support sucré (essai I).

EXEMPLE 12

ESSAIS F à I

Avant de détailler les conditions de réalisation des essais, on précisera les caractéristiques des produits utilisés.


1°) Produits utilisés

- farine : on a utilisé un lot unique de farine. Il s'agit d'une farine de blé de type 55 :

- cendres sur matière sèche ............. 0,53 %
- W (force) ......................... 110 . $10^{-4}$ joules
- protéines ......................... 11,3 %

On a contrôlé que son humidité (14 %) n'a pas évolué tout au long des essais.

- sucroglycérides d'huile de palme commercialisés sous la dénomination CELYNOL MSPO 11

- huile de colza caractérisée précédemment

- sucroglycérides sur support sucré mis sur le marché sous la marque CELYNOL F1

- sucroglycérides de l'invention obtenus selon l'exemple de préparation n° 1.


2°) Conditions de réalisation des mesures

Dans cette série d'exemple et essais, on met en évidence l'action des différentes formes de sucroglycérides sur le gluten par des mesures réalisées au farinographe BRABENDER.

Pour ces tests, on utilise la formule suivante qui est une formule de biscuits type casse-croûte :

- farine ................................ 200 g
- carbonate d'ammonium ...................... 4 g
- sel ...................................... 2 g
- sirop de saccharose à 50 % en poids ........ 106 g
- matière grasse végétale (margarine)......... 15 g
- produit à tester ......................... x

On ajoute les produits à tester suivants dans les quantités (x) ci-après définies :

- essai F : pas d'additif
- essai G : 1,6 g de sucroglycérides
- essai H : 2,4 g d'huile de colza
- exemple 12: 4,0 g de sucroglycérides de l'invention
- essai I : 4,0 g de sucroglycérides sur support sucré.

L'exemple 12 et les essais G et I mettant en oeuvre les sucroglycérides sont réalisés de façon à avoir la même quantité de matière active, soit 0,8 % par rapport à la farine, dose d'emploi moyenne courante dans les industries de cuisson des céréales.

Les mesures sont réalisées à température ambiante constante de 22°C ; l'appareillage et les ingrédients sont maintenus à cette température.

On respectera scrupuleusement le mode opératoire indiqué par les constructeurs du farinographe BRABENDER.

On conduit les mesures au farinographe selon une méthode que l'on ne rappelera que succinctement étant donné qu'elle est bien connue et décrite dans la littérature (cf. Contrôle de la qualité de blé - Guide pratique édité par l'Institut Technique des céréales et des fourrages - Paris, 1972, pages 145-148).

Le farinographe BRABENDER mesure et enregistre l'évolution de la consistance de la pâte au cours d'un pétrissage intensif.

Cet appareil est consitué par un pétrin dont les deux bras fonctionnant en sens inverse (type pétrisseur WERNER) sont actionnés par un moteur dynamométrique qui indique constamment le couple résistant opposé par la pâte au mouvement d'entraînement des bras. A chaque demi-tour des fraseurs, la pâte alternativement comprimée et étirée oppose un couple résistant périodiquement variable oscillant autour d'une valeur moyenne. Un tambour enregistreur synchrone donne un diagramme particulier dit farinogramme qui est une courbe indiquant la variation de la consistance de la pâte exprimée en unités BRABENDER en fonction du temps de pétrissage exprimé en minutes.

On utilise le mode opératoire suivant : on introduit dans la cuve du pétrin, les ingrédients pulvérulents : farine, carbonate d'ammonium, sel puis l'on ajoute le sirop de saccharose et l'on pétrit ensuite 2 minutes à 30 tours/minute. On additionne la matière grasse ainsi que le produit à tester. On reprend le pétrissage pendant 5 minutes à 30 tours/minute.

On exprime les résultats de la manière qui suit :

- consistance de la pâte : il s'agit de la consistance relevée sur l'enregistrement au bout du dernier pétrissage de 5 minutes.

- affaiblissement de la pâte : c'est la différence de consistance entre l'essai témoin et l'essai considéré.

- cohésion de la pâte : elle est représentée par l'épaisseur de la courbe d'enregistrement de la viscosité de la pâte, en fin de pétrissage. Un tracé large indique que la pâte est mal liée et manque d'homogénéité et de cohésion.

Les résultats de l'exemple 12 et des essais sont consignés dans le tableau XII.

Tableau XII

| EX. ESSAIS | Produits à tester | Consistance de la pâte U.B. | Affaiblissement U.B. | Cohésion de la pâte U.B. |
|---|---|---|---|---|
| F | Témoin sans additif | 835 | | 60 |
| G | Sucroglycérides | 780 | 55 | 55 |
| H | Huile de colza | 785 | 50 | 50 |
| 12 | Sucroglycérides de l'invention | 700 | 135 | 45 |
| I | Sucroglycérides sur support sucré | 695 | 140 | 45 |

Il ressort de l'analyse de ce tableau que les sucroglycérides de l'invention et sur support donnent une consistance de la pâte plus faible.

Pour ce qui est de la cohésion de la pâte, elle est également améliorée dans ces deux cas.

La cohésion de la pâte est un point très important dans les industries telles que la biscuiterie sèche ou les pâtes à foncer. Le manque de cohésion amène des tensions internes au cours et après cuisson, ce qui cause des accidents de fabrication tels que la fêle. Un autre avantage est que, la pâte étant plus homogène, on pourra réduire la durée de pétrissage, d'où un gain d'énergie augmenté encore par le fait que la pâte sera moins visqueuse.

On note que les sucroglycérides de l'invention peuvent remplacer sans inconvénient les sucroglycérides sur support sucré dans le domaine de la cuisson des céréales. Ils présentent par rapport à ceux-ci de très gros avantages au niveau de leur mise en oeuvre : facilité de manipulation et meilleure dispersabilité dans l'eau.

REVENDICATIONS

1 - Nouvelle préparation de sucroglycérides sous forme fluide, caractérisée par le fait qu'elle comprend les sucroglycérides et une huile fluide à température ambiante.

2 - Nouvelle préparation selon la revendication 1, caractérisée par le fait que les sucroglycérides mis en oeuvre sont des sucroglycérides d'huile de palme, de suif, de saindoux, d'huile de coprah.

3 - Nouvelle préparation selon l'une des revendications 1 et 2, caractérisée par le fait que l'huile fluide est l'huile de colza, l'huile d'arachide, l'huile de maïs, l'huile de tournesol, l'huile d'oeillette, l'huile de graines de coton, l'huile de soja.

4 - Nouvelle préparation selon l'une des revendications 1 à 3 caractérisée par le fait qu'elle comprend :

- de 30 à 50 % en poids de sucroglycérides
- de 50 à 70 % en poids d'une huile fluide.

5 - Nouvelle préparation selon la revendication 4 caractérisée par le fait qu'elle comprend :

- de 35 à 45 % en poids de sucroglycérides
- de 55 à 65 % en poids d'une huile fluide.

6 - Nouvelle préparation selon la revendication 1 caractérisée par le fait qu'elle comprend une lécithine.

7 - Nouvelle préparation selon la revendication 6 caractérisée par le fait que la lécithine utilisée est de la lécithine de soja ou de la lécithine d'oeuf.

8 - Nouvelle préparation selon l'une des revendications 6 et 7 caractérisée par le fait que la proportion de lécithine représente de 2 à 30 % du poids du mélange constitué par les sucroglycérides et la lécithine.

9 - Nouvelle préparation selon la revendication 8 caractérisée par le fait que la proportion de lécithine représente de 20 à 30 % du poids du mélange constitué par les sucroglycérides et la lécithine.

10 - Nouvelle préparation selon l'une des revendications 6 à 9 caractérisée par le fait qu'elle comprend :

- de 30 à 70 % en poids d'un mélange de sucroglycérides et de lécithine
- de 70 à 30 % en poids d'une huile fluide.

11 - Nouvelle préparation selon la revendication 10 caractérisée par le fait qu'elle comprend :

- de 40 à 60 % en poids d'un mélange de sucroglycérides et de lécithine
- de 60 à 40 % en poids d'une huile fluide.

12 - Nouvelle préparation selon l'une des revendications 6 à 11, caractérisée par le fait qu'elle comprend des sucroglycérides de suif, de la lécithine de soja, de l'huile de soja ou de l'huile de colza.

13 - Procédé d'obtention de la nouvelle préparation de sucroglycérides telle que décrite dans l'une des revendications 1 à 12, caractérisé par le fait qu'il consiste à faire fondre les sucroglycérides, à procéder au mélange des sucroglycérides fondus, de l'huile fluide et éventuellement de la lécithine, à homogénéiser le mélange par une douce et courte agitation qui est ensuite interrompue, à refroidir le mélange obtenu à une température d'au plus 22°C et à le soumettre à une douce agitation.

14 - Procédé selon la revendication 13 caractérisé par le fait que l'on fait fondre les sucroglycérides à une température comprise entre 60 et 80°C.

15 - Procédé selon la revendication 14 caractérisé par le fait que l'on fait fondre les sucroglycérides à une température comprise entre 70 et 75°C.

16 - Procédé selon l'une des revendications 13 à 15 caractérisé par le fait que l'on introduit l'huile à température ambiante de 10 à 25°C progressivement et sous agitation douce dans les sucroglycérides fondus ou inversement.

17 - Procédé selon la revendication 16, caractérisé par le fait que l'on introduit l'huile à température ambiante de 16 à 22°C, progressivement et sous agitation douce dans les sucroglycérides fondus ou inversement.

48

0091331

18 - Procédé selon l'une des revendications 16 et 17 caractérisé par le fait que l'on introduit la lécithine dans les sucroglycérides fondus.

19 - Procédé selon l'une des revendications 16 et 17 caractérisé par le fait que l'on additionne la lécithine simultanément à l'huile.

20 - Procédé selon l'une des revendications 13 à 19, caractérisé par le fait que l'on homogénéise le mélange par une douce agitation de 1 à 15 minutes.

21 - Procédé selon la revendication 20, caractérisé par le fait que l'on homogénéise le mélange par une douce agitation de 2 à 5 minutes.

22 - Procédé selon l'une des revendications 13 à 21, caractérisé par le fait que l'on refroidit le mélange à une température de 15 à 20°C.

23 - Procédé selon l'une des revendications 13 à 22, caractérisé par le fait que l'on soumet le mélange à une agitation de 2 à 15 minutes.

24 - Procédé selon la revendication 23, caractérisé par le fait que l'on soumet le mélange à une agitation de 2 à 5 minutes.

25 - Application de la nouvelle préparation de sucroglycérides sous forme fluide selon l'une des revendications 1 à 12 à la préparation d'émulsions stables de composés hydrophobes dans l'eau tels que les huiles essentielles du domaine alimentaire ; les corps gras alimentaires, margarine, beurre, beurre basses calories , les compositions de laits synthétiques pour l'alimentation des jeunes animaux; les huiles essentielles utilisées en parfumerie et cosmétique ; et les corps gras intervenant dans les différents produits de beauté et d'hygiène ainsi qu'à l'industrie de la cuisson des céréales.

26 - Application selon la revendication 25, caractérisée par le fait que l'on prépare des émulsions stables pouvant contenir jusqu'à 15 à 20 % en poids d'huiles essentielles et la préparation de sucroglycérides de l'invention en une quantité exprimée en sucroglycérides représentant de 40 à 60 % du poids de l'huile essentielle et de l'eau en quantité suffisante pour compléter l'émulsion à 100 % en poids.

27 - Application selon la revendication 26, caractérisée par le fait que l'on prépare des émulsions stables d'huiles essentielles en faisant une dispersion de la préparation de sucroglycérides de l'invention dans l'eau puis en introduisant l'huile essentielle ou en faisant une dispersion d'huile essentielle dans l'eau puis en y ajoutant la préparation de sucroglycérides de l'invention.

28 - Application selon l'une des revendications 26 et 27, caractérisée par le fait que l'huile essentielle est une essence d'orange ou d'anis.

29 - Application selon la revendication 25 caractérisée par le fait que l'on incorpore dans de la poudre de lait de réengraissement pour veaux formé de poudre de lait maigre et de suif, la préparation de sucroglycérides de l'invention telle que décrite dans la revendication 12.

30 - Application selon la revendication 29 caractérisée par le fait que la quantité de préparation de sucroglycérides de l'invention, exprimée en sucroglycérides, représente de 1 à 3 % du poids de suif et que la quantité de suif représente de 15 à 25 % du poids de la poudre de lait.

31 - Application selon l'une des revendications 29 et 30 caractérisée par le fait que l'on introduit la préparation de sucroglycérides de l'invention dans le suif fondu et que l'on sèche simultanément, par atomisation, le mélange obtenu et le lait maigre.

32 - Application selon la revendication 25 caractérisée par le fait que l'on incorpore au cours de la fabrication d'une pâte dans le domaine de la biscuiterie, biscotterie et pâtisserie, la préparation de sucroglycérides de l'invention exprimée en sucroglycérides à raison de 0,3 à 2 % du poids de farine utilisée.

REVENDICATIONS SPECIALES POUR L'AUTRICHE

1 - Procédé d'obtention d'une nouvelle préparation de sucroglycérides sous forme fluide caractérisé par le fait qu'il consiste à faire fondre des sucroglycérides, à procéder au mélange des sucroglycérides fondus et d'une huile fluide à température ambiante, à homogénéiser le mélange par une douce et courte agitation qui est ensuite interrompue, à refroidir le mélange obtenu à une température d'au plus 22°C et à le soumettre à une douce agitation.

2 - Procédé selon la revendication 1, caractérisé par le fait que l'on procède au mélange des sucroglycérides fondus d'une huile fluide à température ambiante et d'une lécithine.

3 - Procédé selon l'une des revendications 1 et 2, caractérisé par le fait que l'on fait fondre les sucroglycérides à une température comprise entre 60 et 80°C.

4 - Procédé selon la revendication 3, caractérisé par le fait que l'on fait fondre les sucroglycérides à une température comprise entre 70 et 75°C.

5 - Procédé selon l'une des revendications 1 à 4, caractérisé par le fait que l'on introduit l'huile à température ambiante de 10 à 25°C progressivement et sous agitation douce dans les sucroglycérides fondus ou inversement.

6 - Procédé selon la revendication 5, caractérisé par le fait que l'on introduit l'huile à température ambiante de 16 à 22°C, progressivement et sous agitation douce dans les sucroglycérides fondus ou inversement.

7 - Procédé selon l'une des revendications 5 et 6, caractérisé par le fait que l'on introduit de la lécithine dans les sucroglycérides fondus.

8 - Procédé selon l'une des revendications 5 et 6, caractérisé par le fait que l'on additionne de la lécithine simultanément à l'huile.

9 - Procédé selon l'une des revendications 1 à 8, caractérisé par le fait que l'on homogénéise le mélange par une douce agitation de 1 à 15 minutes.

10 - Procédé selon la revendication 9, caractérisé par le fait que l'on homogénise le mélange par une douce agitation de 2 à 5 minutes.

11 - Procédé selon l'une des revendications 1 à 10, caractérisé par le fait que l'on refroidit le mélange à une température de 15 à 20°C.

12 - Procédé selon l'une des revendications 1 à 11, caractérisé par le fait que l'on soumet le mélange à une agitation de 2 à 15 minutes.

13 - Procédé selon la revendication 12, caractérisé par le fait que l'on soumet le mélange à une agitation de 2 à 5 minutes.

14 - Procédé selon la revendication 1, caractérisé par le fait que l'on met en oeuvre des sucroglycérides et une huile fluide en une quantité telle que l'on obtienne une préparation ayant la composition suivante :

- de 30 à 50 % en poids de sucroglycérides
- de 50 à 70 % en poids d'une huile fluide.

15 - Procédé selon la revendication 14, caractérisé par le fait que l'on met en oeuvre des sucroglycérides et une huile fluide en une quantité telle que l'on obtienne une préparation ayant la composition suivante :

- de 35 à 45 % en poids de sucroglycérides
- de 55 à 65 % en poids d'une huile fluide.

16 - Procédé selon l'une des revendications 7 et 8, caractérisé par le fait que la proportion de lécithine représente de 2 à 30 % du poids du mélange constitué par les sucroglycérides et la lécithine.

17 - Procédé selon la revendication 16, caractérisé par le fait que la proportion de lécithine représente de 20 à 30 % du poids du mélange constitué par les sucroglycérides et la lécithine.

18 - Procédé selon l'une des revendications 16 et 17, caractérisé par le fait que l'on met en oeuvre des sucroglycérides, une huile fluide et une lécithine en une quantité telle que l'on obtienne une préparation ayant la composition suivante :

- de 30 à 70 % en poids d'un mélange de sucroglycérides et de lécithine
- de 70 à 30 % en poids d'une huile fluide.

19 - Procédé selon la revendication 18, caractérisé par le fait que l'on met en oeuvre des sucroglycérides, une huile fluide et une lécithine en une quantité telle que l'on obtienne une préparation ayant la composition suivante :

- de 40 à 60 % en poids d'un mélange de sucroglycérides et de lécithine
- de 60 à 40 % en poids d'une huile fluide.

20 - Procédé selon l'une des revendications 1 à 19, caractérisé par le fait que les sucroglycérides mis en oeuvre sont des sucroglycérides d'huile de palme, de suif, de saindoux, d'huile de coprah.

21 - Procédé selon l'une des revendications 1 à 20, caractérisé par le fait que l'huile fluide est l'huile de colza, l'huile d'arachide, l'huile de maïs, l'huile de tournesol, l'huile d'oeillette, l'huile de graines de coton, l'huile de soja.

22 - Procédé selon l'une des revendications 2, 7, 8, 16 à 19, caractérisé par le fait que la lécithine utilisée est de la lécithine de soja ou de la lécithine d'oeuf.

23 - Procédé selon l'une des revendications 1 à 22, caractérisé par le fait que les sucroglycérides mis en oeuvre sont des sucroglycérides de suif, l'huile fluide est l'huile de soja, de l'huile de colza, la lécithine utilisée est de la lécithine de soja.

24 - Application de la nouvelle préparation de sucroglycérides sous forme fluide obtenue selon le procédé décrit dans l'une des revendications 1 à 23 à la préparation d'émulsions stables de composés hydrophobes dans l'eau tels que les huiles essentielles du domaine alimentaire ; les corps gras alimentaires, margarine, beurre, beurre basses calories ; les compositions de laits synthétiques pour l'alimentation des jeunes animaux; les huiles essentielles utilisées en parfumerie et cosmétique ; et les corps gras intervenant dans les différents produits de beauté et d'hygiène ainsi qu'à l'industrie de la cuisson des céréales.

25 - Application selon la revendication 24, caractérisée par le fait que l'on prépare des émulsions stables pouvant contenir jusqu'à 15 à 20 % en poids d'huiles essentielles et la préparation de sucroglycérides de l'invention en une quantité exprimée en sucroglycérides représentant de 40 à 60 % du poids de l'huile essentielle et de l'eau en quantité suffisante pour compléter l'émulsion à 100 % en poids.

26 - Application selon la revendication 25, caractérisée par le fait que l'on prépare des émulsions stables d'huiles essentielles en faisant une dispersion de la préparation de sucroglycérides de l'invention dans l'eau puis en introduisant l'huile essentielle ou en faisant une dispersion d'huile essentielle dans l'eau puis en y ajoutant la préparation de sucroglycérides de l'invention.

27 - Application selon l'une des revendications 25 et 26, caractérisée par le fait que l'huile essentielle est une essence d'orange ou d'anis.

28 - Application selon la revendication 24, caractérisée par le fait que l'on incorpore dans de la poudre de lait de réengraissement pour veaux formé de poudre de lait maigre et de suif, la préparation de sucroglycérides de l'invention obtenue selon le procédé décrit dans la revendication 23.

29 - Application selon la revendication 28, caractérisée par le fait que la quantité de préparation de sucroglycérides de l'invention, exprimée en sucroglycérides, représente de 1 à 3 % du poids de suif et que la quantité de suif représente de 15 à 25 % du poids de la poudre de lait.

30 - Application selon l'une des revendications 28 et 29, caractérisée par le fait que l'on introduit la préparation de sucroglycérides de l'invention dans le suif fondu et que l'on sèche simultanément, par atomisation, le mélange obtenu et le lait maigre.

31 - Application selon la revendication 24, caractérisée par le fait que l'on incorpore au cours de la fabrication d'une pâte dans le domaine de la biscuiterie, biscotterie et pâtisserie, la préparation de sucroglycérides de l'invention exprimée en sucroglycérides à raison de 0,3 à 2 % du poids de farine utilisée.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | CHEMICAL ABSTRACTS, vol. 91, 1979, page 501, no. 37798w, Columbus, Ohio, USA & JP - A - 79 32511 (DAIICHI KOGYO SEIYAKU CO., LTD.) 09-03-1979 * Résumé * | 1-3,6-9,12-14 | A 23 D 3/00<br>A 23 D 5/00<br>A 21 D 2/16<br>A 61 K 7/00<br>B 01 F 3/08<br>A 23 L 1/22<br>A 23 C 15/16<br>A 23 C 11/04 |
| X | US-A-3 914 458 (K. TERADA et al.) <br><br>* Revendication 1; colonne 2, lignes 63-67; colonne 3, lignes 1-22, 27-33 * | 1-3,13-15,22,25,28 | |
| A | CHEMICAL ABSTRACTS, vol. 95, 1981, page 342, no. 103141n, Columbus, Ohio, USA & JP - A - 81 55306 (SHISEIDO CO., LTD.) 15-05-1981 * Résumé * | 1,25 | |
| A | EP-A-0 005 290 (PROCTER & GAMBLE) <br>* Revendications 1,3,5; page 8, lignes 11-18 * | 1,6,7,25 | DOMAINES TECHNIQUES RECHERCHES (Int. Cl. 3)<br><br>A 23 D<br>A 21 D<br>A 23 C<br>A 23 L |
| A | FR-A-2 483 455 (M. VOISIN) <br><br>* Revendications 1,7,8; page 2, alinéa 4; page 3, alinéa 4; exemples 1,2 * | 1,6,25,28 | |

--- -/-

Le présent rapport de recherche a été établi pour toutes les revendications

| Lieu de la recherche | Date d'achèvement de la recherche | Examinateur |
|---|---|---|
| LA HAYE | 21-06-1983 | PEETERS J.C. |

Office européen
des brevets

**RAPPORT DE RECHERCHE EUROPEENNE**

**0091331**
Numéro de la demande

EP 83 40 0473

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int. Cl. ³) |
|---|---|---|---|
| A | CHEMICAL ABSTRACTS, vol. 72, 1970, page 106, no. 45246n, Columbus, Ohio, USA R. COLSON: "Use of sucrose esters and sucroglycerides in cosmetics, pharmaceutical excipients, and in the food and agricultural industries" & CHEM. PHYS. APPL. SURFACE ACTIVE SUBST., PROC. INT. CONGR., 4th 1964 (Pub. 1967), 3, 495-504 * Résumé * | 1,29 | |
| A | --- FOOD TECHNOLOGY, vol. 28, no. 10, octobre 1974, page 111 & JP - A - 74 21072 (MORINAGA MILK IND. CO., LTD.)* Page 111, colonne 2, alinéa 3 * ----- | 1,29 | |

**DOMAINES TECHNIQUES RECHERCHES (Int. Cl. ³)**

Le présent rapport de recherche a été établi pour toutes les revendications

| Lieu de la recherche | Date d'achèvement de la recherche | Examinateur |
|---|---|---|
| LA HAYE | 21-06-1983 | PEETERS J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03 82